# EUROPEAN PATENT SPECIFICATION

(11) **EP 3 597 248 B1**
(45) Date of publication and mention of the grant of the patent: **30.08.2023**
(21) Application number: 19190874.8
(22) Date of filing: 10.12.2012
(51) Int. Cl.: A61M 16/06, A61M 16/08, A61M 16/12, A61M 16/20, A61B 18/10

(54) **VALVE FOR RESPIRATORY MASKS**
VENTIL FÜR ATEMMASKEN
SOUPAPE DESTINÉE À DES MASQUES RESPIRATOIRES

(30) Priority: 09.12.2011 GB 201121192
(43) Date of publication of application: 22.01.2020
(62) Divisional of application: 12196351.6
(73) Proprietor: Intersurgical AG, 9490 Vaduz (LI)
(72) Inventor: BOWSHER, Richard Francis, Reading, Berkshire RG31 7DB (GB)
(74) Representative: Adamson Jones

(56) References cited:
- EP-A1- 1 854 494
- WO-A1-2011/121472
- US-A- 4 538 620
- US-A1- 2010 263 669

## Description

This invention relates to valves for respiratory masks and, more particularly, although not exclusively, to valves of a type which may be mounted in a wall of the respiratory mask for communication with ambient air.

Simple respiratory masks generally fit over a patient's nose and mouth, and are secured around the patient's head by an elastic strap. Gas is delivered through a tube connected to the base of the mask, and openings on each side of the mask enable the escape of exhaled gases.

For circumstances in which it is necessary to control the concentration of gases delivered to the patient it is known to provide valves in the side of the mask instead of simple openings. Respiratory masks of this kind may be used for oxygen therapy in which oxygen is administered to a patient at a concentration greater than that of ambient air, and/or aerosol therapy in which a fine mist of a drug in solution is inhaled by a patient. Some embodiments of such masks are often referred to as "high concentration" masks.

The valves ensure that the inspiratory flow to the patient is provided via the gas delivery tube rather than being entrained through the openings in the mask wall. The valves are often referred to as "exhalation valves" since they allow expired gas to exit the mask. High concentration respiratory masks generally include a reservoir bag that is charged with gas during use, so that sufficient respiratory gas, is available to meet most peak inspiratory flow demands of the patient.

Conventional exhalation valves typically comprise an annular or disk-like member which is held against a valve seat and which deforms such that the entire periphery of the valve member lifts off the valve seat in response to a sufficient pressure differential across the valve to thereby allow passage of gas between the valve member and valve seat.

EP 1 854 494, for example, discloses a respiratory mask with a plurality of separate ports for enabling passage of gas into, and out of, the cavity of the mask body, and a valve assembly. The valve assembly includes a continuous valve membrane having a deformable first valve member that controls flow of gas through a first port, and an independently deformable second valve member that controls the flow of gas through a second port.

Whilst this type of exhalation valve is simple in construction, its operation is reliant upon the resilience of the valve member, which can restrict the available range of movement of the valve. Thus the valve member resists opening and can restrict the available outlet flow area through the valve, thereby preventing a portion of the expired gas from exiting the mask during each breathing cycle and increasing the resistance to flow and work of breathing. This is undesirable for non-rebreathing masks.

Developments in mask technology have resulted in improved seals between the mask and the patient's face such that little, or no, ambient air can be entrained into the mask about the mask periphery during inspiration. Whilst this is beneficial in ensuring the desired concentration of gas delivered to the patient is maintained, such improved sealing creates an increased risk of asphyxiation since, in the event that gas supply via the tubing is inhibited, the patient may be unable to draw sufficient breath. This is a particular problem if the patient is unconscious or incapacitated to the extent that they cannot remove the mask by hand. It is known to provide a safety, anti-asphyxiation, valve in the mask body that can allow ambient air into the mask in the event that the gas supply is insufficient to meet the patient's inspiratory demand.

Known valves of this kind are often more complex in construction than exhalation valves since they are intended to be inoperative during normal use of the mask so as to prevent entrainment of ambient air into the gas delivered to the patient. Furthermore, since those valves typically open inwardly to allow air into the mask, it is often difficult to see whether the valve is open.

It is an aim of the present invention to provide an improved valve construction that can better accommodate the requirements of an exhalation valve and/or also an anti-asphyxiation valve.

According to the present invention there is provided a respiratory mask as defined in the appended claim 1. Further optional features are defined in the associated dependent claims.

Also described is a valve for a respiratory mask, the valve comprising a valve member and a valve body, the valve member being actuable between open and closed conditions relative to the valve body in response to a pressure differential across the valve, wherein the valve member comprises first and second portions arranged for contact with the valve body in the closed condition and the valve member is pivotably mounted to the valve body between the first and second portions such that the first and second portions of the valve member are actuated into the open condition in opposing directions.

The valve member may be loosely mounted on the valve body such that the valve member can tilt relative to the valve body. The valve body may comprise a support formation which may comprise a projection or stem. The valve member may comprising an opening through which the projection extends. The projection may comprise a neck and a head formation, wherein the neck may have a width dimension which is less than that of the opening and the head may have a width which is greater than that of the opening. The neck is preferably longer than the thickness of the valve member such that the valve member is loosely positioned about the neck.

The tilting action of the valve member is particularly advantageous in that it requires a smaller pressure difference across the valve member in order to actuate the valve between closed and open conditions. The deformation of the valve member between closed and open conditions is reduced. Accordingly the expired gas flow does not need to work against the resilience of the valve member to exit the mask.

The tilting action of the valve member is advantageous also in that it can more-readily open to a wider degree than conventional valves which are deformed between open and closed conditions. Thus for a common valve size, the present invention may allow a greater expiratory flow rate therethrough and/or a reduced resistance to flow and a reduced work of breathing.

The tilting action is further beneficial in that it can provide a readily visible indicator of the actuation of the valve. Thus a carer can see at a glance whether the valve is being actuated and thereby deduce whether a patient is breathing correctly. A carer may also readily deduce the rate of breathing.

The support formation may comprise a cross member extending at least part way across a valve opening within the valve body. The valve member may be arranged to pivot about the cross member. The projection may depend from the cross member. The projection may be upstanding from the cross member.

The first and second portions of the valve member may comprise opposing halves of the valve member. The cross member may be arranged to extend across a mid line of the valve member, for example at the interface between the first and second valve member portions.

The valve member may be mounted to the valve body between the first and second portions, for example about its mid-point. The valve member may have a generally circular peripheral edge. The valve member may be annular in form.

The first portion of the valve member may be actuated outwardly of the mask body between the closed condition and the open condition. The second portion may be actuated inwardly of the mask body between the closed condition and the open condition.

The valve body may comprise first and second contact surfaces for contact with the respective first and second portions of the valve member. The first and second contact surface may face in opposite directions. The first and second contact surfaces may be provided on opposing sides of a common wall. The valve body may be formed in a wall of a mask. The valve body may be integral with the mask body.

In a respiratory mask according to the invention, the second contact surface is offset from the first contact surface such that the valve member is deformed in its closed condition. For example the valve member may lie slightly ajar from the first contact surface in an at-rest condition and may be pressed against the first contact surface only upon application of a pressure gradient across the valve, which pressure gradient is typically a slight pressure gradient, such as a slight negative pressure on a patient side of the valve, which may be induced by a patient drawing breath.

The second contact surface may have a greater contact area than the first contact surface. The first contact surface may be shaped to contact the valve body in the vicinity of its peripheral edge. The second contact surface may cover an area of the second portion of the valve member in the closed condition. The second contact surface may overlie for example between a quarter and a half of the second portion of the valve member. In this manner the valve member is prevented from opening or being actuated in a reverse direction.

The valve body may be shaped to define a valve opening. The valve opening may be asymmetrical with respect to the valve member and/or support formation. Thus when the valve member tilts to the open condition, the opening may be divided by the valve member into first and second portions of different area. The first portion of the opening may provide a greater flow area than the second portion of the opening. The first and second opening portions may correspond to the first and second valve member portions.

The valve member and/or support formation may be arranged such that the valve member pivots into the open condition to allow escape of expired air in a direction away from a wearer of the mask. The pivoting action of the valve member thus allows a more directional outlet flow from the mask. This can prevent discomfort to a patient, particularly over an extended period of use of the mask.

The valve member may comprise a resilient and/or flexible material.

The open condition of the valve may be a first open condition. The valve may have first and second open conditions. The valve may be a bi-directional valve. The valve may act as both an exhalation valve and also a safety valve, for example by allowing for different modes of actuation of a single valve member. The valve member may be actuated in the different modes of operation by application of positive and negative pressures across the valve member.

The valve member may be actuated into the first open condition by tilting, for example upon application of a positive pressure on a first side, or patient side, of the valve. The valve member may be actuated in the second open condition by resilient deformation of the second valve portion in to an open condition, for example whilst the first valve portion remains in a closed condition. The second open condition may be achieved by application of a negative pressure on the first side of the valve (or else by application of a positive pressure on the second, or ambient, side of the valve).

The valve member may resiliently deform into the second open condition such that the valve member is biased against gas flow therethrough. The magnitude of the pressure differential required to operate the valve in the second condition is preferably greater than the magnitude of the pressure differential required to operate the valve member in the first open condition. This may be because the valve member can pivot freely in a first direction into the first open condition from a closed condition, whereas the valve member must be deformed against the resilience of the valve member material in the second open condition.

The valve body may be shaped such that a greater surface area of the first portion of the valve member is presented to the exterior of the mask body than that of the second portion.

The respiratory mask of the invention has a valve body therein shaped to define an opening between the interior and exterior sides of the mask, the valve body supporting a valve member which is actuable between open and closed conditions relative to the valve body in response to a pressure differential across the valve member, wherein the valve member is openable in both a first mode of operation in response to a positive relative pressure on the interior side to allow escape of gas from the mask and a second mode of operation in response to a negative relative pressure on the interior of the mask to allow gas to be drawn into the mask from the exterior side. The valve member is substantially undeformed in said first mode of operation. A first portion of the valve member is arranged to contact a lip of the valve body and a second portion of the valve member is arranged to contact a formation offset from the lip in its closed condition such that the valve member is deformed in said closed condition.

In the first mode of operation, the valve member may open outwardly of the mask body, for example by tilting or pivoting. In the second mode of operation the valve member may open by deformation of the valve member, for example only inwardly of the mask body. The provision of this dual functionality at a location which is spaced from a conventional inlet valve in the mask is advantageous in that it can serve to reduce manufacturing and/or operational complexity. The valve also provides a clear visual indication of respiration and/or the adequacy of the respiratory supply.

Also described is a respiratory mask having a valve body therein shaped to define an opening between the interior and exterior sides of the mask, the valve body supporting a valve member which is actuable between open and closed conditions relative to the valve body in response to a pressure differential across the valve member, wherein the valve member comprises first and second portions arranged for contact with the valve body in the closed condition and the valve member is pivotably mounted to the valve body at a location between the first and second portions such that the first and second portions of the valve member are actuated into the open condition in opposing directions.

The valve body may be integral with the mask, such as for example, within a wall of the mask. The mask may be generally rigid in form and may be generally concave in shape. The valve body may be formed in a side wall portion of the mask. A pair of valve bodies and associated valve members may be provided in the mask body, typically in opposing side wall portions of the mask. Those side wall portions may be arranged on either side of a wearer's nose in use.

Any of the optional features defined above in relation to valves may also be applied to respiratory masks according to the invention.

Practicable embodiments of the invention are described in further detail below with reference to the accompanying drawings, of which:
Figure 1 shows a three-dimensional view of a mask according to one example of the invention;
Figure 2 shows a perspective view of the valve member of Figure 1;
Figure 3 shows a plan view of the valve body of Figure 1;
Figure 4 shows a section view of the valve body of Figure 3 taken through the plane A-A';
Figure 5 shows a section view of the valve body of Figure 3 taken through the plane B-B';
Figure 6 shows a section of an example valve in a closed condition;
Figure 7 shows a section view of the valve of Figure 6 in a first open condition; and,
Figure 8 shows a section view of the valve of Figure 6 in a second open condition.

The present invention derives from the realisation by the inventor that a conventional expiration valve in the side wall of a respiratory mask can be modified to accommodate the function of an anti-asphyxiation - or safety - valve. By modifying the valve function, further surprising functional advantages have become apparent as will be described below.

It will be appreciated that respiratory masks and valves therein are safety-critical.

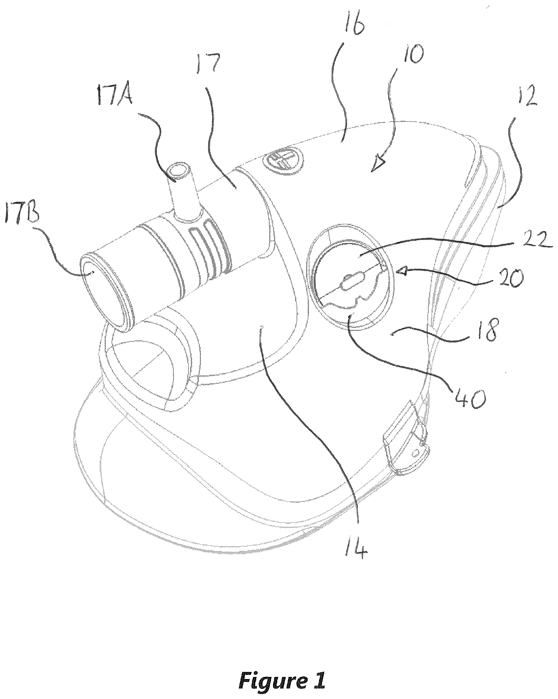

Turning firstly to Figure 1, there is shown an example of a respiratory mask according to the present invention, which is suitable for the delivery of a gas to a wearer, such as a patient. The respiratory mask comprises a mask body 10, formed from a suitably strong and relatively rigid plastics material, such as polypropylene, and a sealing formation 12 about the periphery of the mask formed from a softer material which can conform to the contours of a wearer's face. The seal material in this example is moulded onto the mask body and may comprise an elastomer such as a Styrene-Ethylene-Butylene-Styrene (SEBS)-based thermoplastic elastomer.

The mask body 10 is generally concave, so as to define a cavity to which respiratory gas is delivered for inhalation by a wearer. The mask body 10 comprises a mouth-accommodating portion 14 and a nose-accommodating portion 16. The mask body is shaped such that the depth of the cavity defined by the nose portion is greater than the depth of the cavity defined by the mouth portion.

An inlet connector formation 17 is provided at the mask wall portion between the nose and mouth sections of the mask. In use the inlet connector is attached at connector formation 17A to a respiratory gas delivery tube (not shown), which is typically connected to a compressed respiratory gas source, such as an oxygen supply. The inlet comprises an inlet valve for controlling the delivery of gas to the patient. The inlet connector 17 may also allow connection at port 17B of a conventional reservoir bag (not shown) as are typically used with high concentration respiratory masks. Such a bag is charged with respiratory gas, such as oxygen, from the gas supply in use, so that sufficient gas is readily available to meet peak inspiratory flow demands of the patient.

Side walls 18 of the mask body extend downwards from the nose portion 16 on either side of the mouth portion 14. A valve assembly 20 according to the present invention is formed in each of said side walls 18, the form and function of which will be described below. However it will be appreciated that one or more valve assemblies 20 could be formed at any suitable location in the wall of the mask body such that it is spaced from, and distinct in function from, the inlet valve. It is of note that in any such location, the valve assembly 20, and more specifically the actuable valve member 22 of the valve, is exposed and thus visible from outside of the mask, for example to a carer.

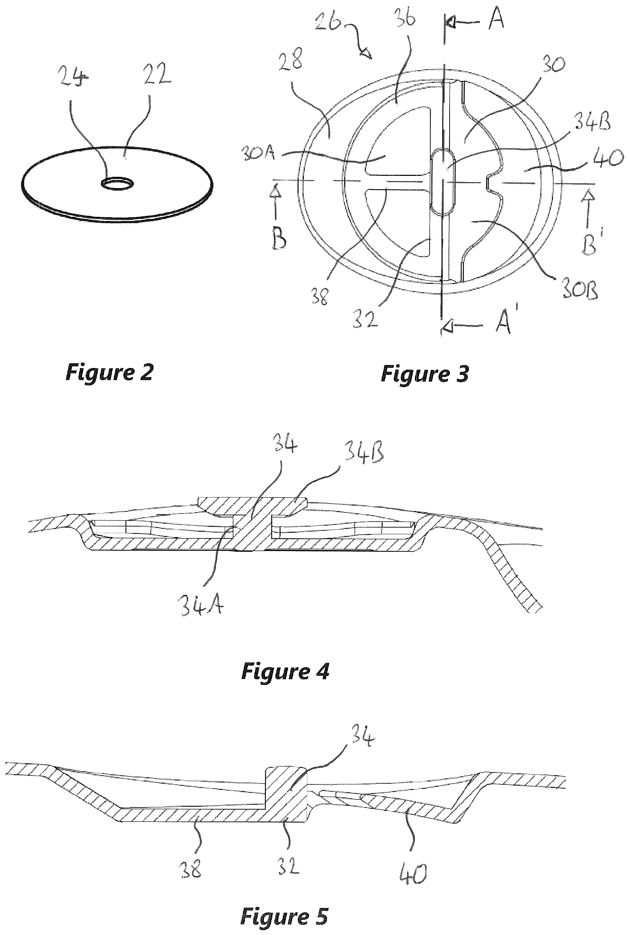

Turning now to Figure 2, there is shown the valve member 22, which is mounted to the valve body of Figure 3 for use. The valve member 22 is annular in form and comprises a singular body of resilient or flexible material such as silicon. The valve member has a central opening 24 by which it is mounted the valve body 26 shown in Figures 3 to 5. The valve member is flat or planar in an at-rest or un-deformed condition as shown in Figure 3.

In Figure 3, there is shown a valve body 26 of the valve assembly 20 of Figure 1. Although the valve body 26 is shown in isolation of the remainder of the mask body in Figure 3, the valve body in this embodiment is formed integrally with the mask body, for example as a single moulded component.

The valve body 26 comprises a peripheral wall 28 which depends inwardly from the wall of the mask body. The peripheral wall 28 defines a substantially circular opening 30 in the wall of the mask. In this embodiment the opening 30 is sunken or recessed slightly with respect to the outer wall of the mask body 10 by virtue of the depth of the peripheral wall 28.

The opening 30 defines a gas flow port in which the valve member 22 is located in use. In this regard, the valve body 26 comprises support formations for the valve member 22. The support formations comprise a support bar 32, which takes the form of a strut or beam. The support bar 32 extends across the diameter of the opening 30 and has formed thereon an upstanding stem 34, as can be seen in section in Figure 4.

The stem 34 has a neck portion 34A of width smaller than or substantially equal to the width of the opening 24 of the valve member 22. Preferably the width of the stem neck 34A is smaller than that of the opening 24 so as to allow the valve member to be loosely seated about the stem neck.

The stem 34 has an enlarged head formation 34B which has a width dimension that is greater than the diameter of the opening 24 in the valve member 22. Thus, once the valve member 22 is mounted on the stem 34 about the neck 34A, it is trapped between the head formation 34B and the cross bar 32. The depth of the neck 34A is greater than the thickness of the valve member such that the valve member is loosely held against the valve body and can ride a short distance up and down the valve stem in use. The enlarged head formation 34B has a chamfered edge to aid assembly of the valve member 22.

It is notable that the head formation 34B is elongate in the direction of the cross bar 32, rather than being rotund in form, such that the stem 34 takes the form of a T-shaped structure. This both permits assembly of the valve member over the stem 34 and also promotes tilting of the valve member about the cross bar 32 in use as will be described below.

Also visible in Figure 3 is a peripheral lip formation 36 which extends part way around the opening 30. The lip 36 depends inwardly into the opening 30 from the adjacent wall 28 and thereby reduces the area of the opening. The lip is semi-annular in plan. In this embodiment the lip 36 is integrally formed with the cross bar 32 and lies in the same plane as the cross bar.

Also provided is a support arm 38 extending from the cross bar in the vicinity of the stem 34 to the lip 36. As can be seen in Figure 5, the arm 38, the lip 36 and the cross bar 32 lie in the same plane and provide a common support structure for the valve member 22 in the closed position. Accordingly, any or any combination of the cross bar 32, lip 36 and/or support arm 38 is referred to below as a valve seat.

The valve seat lies within one half 30A of the opening 30 (i.e. the left hand side of the opening as shown in Figure 3). In the opposing half 30B of the opening 30, there is provided a further formation 40, which is arranged to contact the valve member 22 in use as will be described below. The formation 40 depends inwardly from the periphery of the opening 30 in half 30B in a manner similar to the lip formation 36. In this embodiment the formation 40 has a larger surface area than the lip 36 and thus covers or spans a greater area of the opening 30.

Also as can be seen in Figure 5, the formation 40 is angled with respect to the cross bar 32 and lip 36 such that it depends out of the plane in which those members are located. The formation 40 is generally crescent shaped in plan. In Figure 3 it can be seen that the formation 40 also has a central protrusion, which is generally radially aligned such that it protrudes from the inner edge of the formation a short way towards the centre of the opening. This protrusion helps to prevent the valve from inverting and catching on the valve seat.

As can be seen in Figure 1, the formation 40 lies towards the peripheral edge and seal 12 of the mask body 10. Accordingly the formation 40 can be considered to be arranged to the rear of the lip 36. In this manner the lip 36 is arranged towards the front of the mask, that is away from the wearer of the mask in use. The half 30A of opening 30 is provided towards the front of the mask, whilst the opposing half 30B is located towards the rear (i.e. towards the wearer in use).

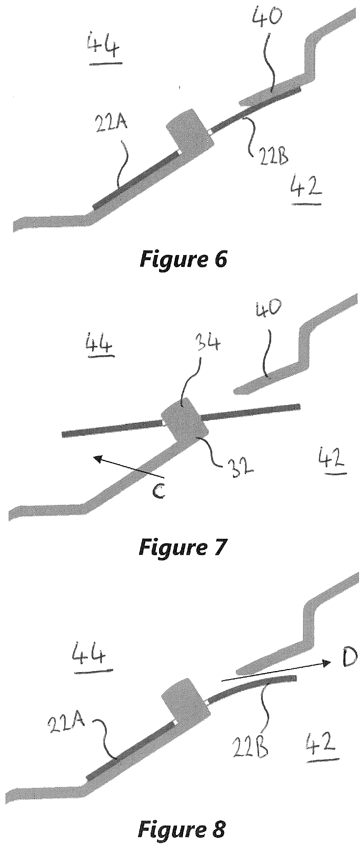

Turning now to Figure 6, there is shown a section view of the valve 20 in its closed condition. The patient side of the valve is designated 42, whereas the exterior or the valve/mask is designated 44. The exterior 44 is at ambient pressure, which will typically be approximately equal to atmospheric pressure in normal use.

In Figure 6, it can be seen that the formation 40 is chamfered at its inner edge and rounded on the side remote from the patient, in use. The valve seat and also the formation 40 each have a substantially constant thickness which is similar to that of the mask wall. The valve body formations are all suitable for moulding integrally with the mask body as a single piece.

The valve member 22 has a first side which faces the interior of the mask 42 and a second side which faces the exterior of the mask 44. When mounted on the stem, the valve member 22 is effectively divided into two halves, indicated as 22A and 22B, which selectively cooperate with the valve body 26 on opposing halves 30A and 30B of the opening 30 respectively. Valve member portion 22A is arranged to contact the lip 36, whereas valve member portion 22B is arranged to contact the formation 40 in its closed condition. As can be seen in Figure 6, the interior face of the valve member 22 contacts the lip 36 whereas the exterior face of the valve member 22 contacts the formation 40.

In the closed condition, the valve member portion 22A lies flat against the cross bar 32 and lip 36 so as to block the opening portion 30A and thereby prevent flow into the mask from the exterior 44. The opposing face of the other half 22B of the valve member 22 contacts the formation 40 such that the half 30B of the opening 30 is also closed. Thus the valve member 22 abuts both the valve seat and opposing formation 40.

It is to be noted in this closed condition, that the offset between the lip 36 and the formation 40 causes a slight deflection in the valve member 22 such that the valve member is deformed in the closed condition. Accordingly, when at rest, the valve member would naturally contact the formation 40 on side 22B but would lie slightly ajar or open on the side 22A.

The application of a slight negative pressure on the patient side 42 of the valve, for example caused by the wearer drawing breath, is sufficient to deform the valve member 22 into the closed condition shown in Figure 6. This condition serves to ensure that, when the gas supplied to the mask via connector 17 is sufficient to meet the wearer's inspiratory demand, the valve adopts the closed condition so as to ensure that the patient does not inspire ambient air. This serves to ensure that the desired concentration of respiratory gas is maintained throughout normal use of the mask.

During expiration, as gas from the wearer's lungs is expelled into the interior cavity of the mask, the fluid pressure on the interior side 42 of the valve increases above ambient, which applies a force on the portion 22A of the valve member and thereby causes the valve member to pivot on the cross bar 32 into a first open condition. This pivoting of the valve member 22 is permitted by the loose fitment of the valve member 22 on the stem 34 such that the valve member 22 is substantially undeformed in the first open condition. The head formation 34B on the valve stem 34 may have an angled, for example, chamfered, surface which faces the valve member in order to encourage this tilting action

The tilting of the valve member in this manner opens both halves 30A and 30B of the opening 30. Thus the single opening 30 is effectively divided by the valve member 22 into two flow openings 30A and 30B on each side of the cross bar 32. Gas is expelled from the mask via the valve opening in the direction of arrow C, so as to resolve the pressure difference between the interior and exterior of the mask.

The presence of the formation 40 and lip 36 on opposing sides of the mask will act as stops so as to prevent unwanted pivoting of the valve in an incorrect direction. Thus the valve member reliably pivots (e.g. in the clockwise direction as shown in Figure 7) into the open condition upon application of a slight positive pressure inside the mask 42. The larger area of the formation 40, compared to that of the lip 36, causes a greater blockage or obstruction on the side 30B of the opening than on side 30A. This difference in flow area may also serve to promote correct opening of the valve, although this is may not be essential to the operation of the valve.

Once the ambient and internal pressures have equalised the valve member 22 will remain at rest until a negative pressure is again applied during inspiration. Thus the valve member alternates between the first open condition and the closed condition during normal use. The opening of the valve in a pivoting manner in the first open condition as described above is beneficial since the direction of the flow of gas exiting the mask can be controlled. In this embodiment, the valve body 26 and valve member 22 are oriented such that the valve opens in a direction away from the wearer. Thus the fluid flow exiting the mask is directed away from the face of the wearer. This directional property of the valve can avoid discomfort during use of the mask, for example, due to gas flow irritating a wearer's eyes.

Furthermore the tilting actuation of the valve member provides a pronounced angular movement which is readily visible to an onlooker, regardless of their position relative to the mask. For example, the marked change in orientation of the valve member 22 between open and closed conditions can be seen at a glance from either side, from above or below, or else from in front of the mask wearer.

The range of possible movement of the valve also allows the valve member 22 to open to an angle such that it provides little resistance to the escape of air from the mask once open.

The angle of movement of the valve member 22 between the first open condition and the closed condition may be for example between 20° and 45°. That angle may typically be between 25° and 40°.

Turning now to Figure 8, there is shown a further open condition of the valve. Here it can be seen that the portion 22A of the valve member 22 is closed but that the portion 22B is deformed into an open condition. The portion 22B in this condition has flexed such that it is spaced from the formation 40. In this regard, the portion 22B has deformed into the interior of the mask body.

The second open condition is achieved in response to the presence of a negative pressure gradient across the valve at or above a predetermined magnitude. That is to say if the pressure on the inside of the mask 42 is lower that the external pressure by only a small amount, then the resilience of the valve member 22 will hold the valve closed against such a pressure difference. However the increase in the pressure difference between the interior 42 and exterior 44 of the mask above a predetermined magnitude (i.e. by lowering the internal pressure) will exert a force on the valve member 22 which is sufficient to deform the portion 22B into the open condition shown in Figure 8. Such a negative pressure may be achieved in practice by a user attempting to draw breath at a rate greater than that the supply of gas via the mask inlet at connector 17.

In the condition shown in Figure 8, a negative pressure on the internal side 42 urges the portion 22A closed against the valve seat but simultaneously urges the portion 22B open. In this manner ambient air can be drawn into the mask and inhaled by a wearer in the event that the gas supply is insufficient. Accordingly the portion 22B of the valve member 22 acts as a safety valve in the event of a blockage, disconnection or other malfunction of the gas supply such that a wearer can draw sufficient air through the valve without having to remove the mask 10.

It is noted that this second mode of operation, as a safety valve, relies on the deformation of the valve member 22 in contrast to the tilting operation of the valve during normal use. In this regard the valve member 22 material and surface area as well as the offset of the formation 40 relative to the valve seat are important considerations which determine the threshold pressure difference required to open the valve in the second open condition. Of those factors, it has been found that it is possible to achieve the desired operation of the valve by using conventional valve materials and by amending the offset and area of formation 40 only.

The formation 40 is arranged such that the valve member 22 is deflected in its closed condition by approximately between 1 and 20° and more preferably by between 2° or 5° and 10°.

The threshold pressure difference required to open the valve member 22 in its second open condition is beneficial in that it avoids the valve opening to satisfy *de-minimis* pressure fluctuations which may be transient in nature. The threshold pressure, and thereby the sensitivity of the valve to negative pressures, can be adjusted by altering the resilience of the valve member 22, for example by altering the thickness or material of the valve member 22, or else by adjusting the offset or size of the formation 40.

In view of the above, it will be appreciated that the invention allows for the provision of a valve which can accommodate the function of both an exhalation and a safety valve by arranging the valve member 22 such that it divides the flow opening of the valve into two sections which can function differently under varying pressure and/or flow conditions. Whilst the above example is described as having a circular valve opening 30 and valve member 22, such that each opening section is generally semi-circular, it will be appreciated that other configurations are possible, in which the valve may be for example elliptical or polygonal and/or in which the valve member 22 may be pivoted about an axis which is offset from a centreline of the opening 30.

Also described herein is a valve for a respiratory mask, the valve comprising a valve body shaped to define a valve opening and a valve member which is mounted to the valve body such that the valve member is actuable between open and closed conditions relative to the valve body in response to a pressure differential across the valve, wherein the valve member comprises first and second portions arranged for contact with the valve body in the closed condition and the valve member is pivotably mounted such that the first and second portions of the valve member are actuated into the open condition in opposing directions relative to the valve body.

The valve member may be mounted to the valve body at a location between the first and second portions.

The first and second portions may comprise opposing sides or ends of the valve member.

The valve member may comprise a flexible material and may be loosely mounted to the valve body such that the valve member can tilt relative to the valve body substantially without deformation of the valve member.

The valve body may comprise a support formation having a cross member which extends at least part way across the valve opening and the valve member may be pivotably mounted with respect to the cross member.

The valve body may comprise first and second contact formations for contact with the respective first and second portions of the valve member in the closed condition, wherein the first and second contact formations are arranged to contact opposing faces of the valve member.

At least one of the first and second contact formations may be shaped to partially cover the valve opening so as to define flow areas of different size with respect to the first and second portions of the valve member in the open condition.

The open condition may comprise a first open condition in which the valve member opens in response to a positive pressure gradient across the valve and a second open position, in which only the second portion of the valve member opens in response to a negative pressure across the valve.

The valve member may be resiliently deformable and may deform in response to the negative pressure in the second portion in order to allow flow there-through.

The magnitude of the positive pressure gradient required to actuate the valve to the first open condition may be less than the magnitude of the negative pressure gradient required to actuate the valve into the second open condition.

The valve body may be integrally formed with a wall of a respiratory mask.

A respiratory mask may comprise a valve as previously described, where the valve body is provided in a wall of the mask.

## Claims

1. A respiratory mask (10) having a valve body (26) therein shaped to define an opening (30) between interior and exterior sides of the mask (10), the exterior side of the mask (10) being open to ambient conditions, the valve body (26) supporting a deformable valve member (22) which is actuable between open and closed conditions relative to the valve body (26) in response to a pressure differential across the valve member (22),
wherein the valve member (22) is openable in both a first mode of operation in response to a positive relative pressure on the interior side to allow escape of gas from the mask (10) to the exterior of the mask (10) and a second mode of operation in response to a negative relative pressure on the interior of the mask (10) to allow gas to be drawn into the mask (10) from the exterior side;
**characterised in that** the valve member (22) is substantially undeformed in said first mode of operation, and **in that** a first portion (22A) of the valve member (22) is arranged to contact a lip (36) of the valve body and a second portion (22B) of the valve member (22) is arranged to contact a formation (40) offset from the lip (36) in its closed condition such that the valve member (22) is deformed in said closed condition.

2. A respiratory mask (10) according to any of claim 1, wherein the valve member (22) is a singular body of resilient or flexible material.

3. A respiratory mask (10) according to claim 1 or 2, wherein the valve member (22) is annular in form.

4. A respiratory mask (10) according to any preceding claim, wherein the valve member (22) is flat or planar in an at-rest or un-deformed condition.

5. A respiratory mask (10) according to any preceding claim, comprising a connector formation (17) for connection to a gas supply, the connector formation (17) comprising an inlet into the mask (10), wherein the valve body (26) is spaced from the inlet.

6. A respiratory mask (10) according to any preceding claim, wherein the valve body (26) is provided in a side wall (18) of the mask (10).

7. A respiratory mask (10) according to any preceding claim, wherein the valve member (22) opens outwardly of the mask body (10) in the first mode of operation.

8. A respiratory mask (10) according to any preceding claim, wherein the valve member (22) is visible from outside of the mask (10).

9. A respiratory mask (10) according to any preceding claim, wherein the valve member (22) is deformed in the second mode of operation.

10. A respiratory mask (10) according to claim 9, wherein the valve member (22) is deformed inwardly of the mask body (10) in the second mode of operation.

11. A respiratory mask (10) according to any preceding claim, wherein only the second portion (22B) of the valve member (22) opens in the second mode of operation.

12. A respiratory mask (10) according to Claim 11, wherein the valve member (22) deforms in response to the negative pressure in the second portion (22B) in order to allow flow there-through.

13. A respiratory mask (10) according to any preceding claim, wherein the magnitude of the positive pressure gradient required to open the valve (20) in the first mode of operation is less than the magnitude of the negative pressure gradient required to open the valve (20) in the second mode of operation.

## Patentansprüche

1. Atemmaske (10) mit einem Ventilkörper (26) darin, der so geformt ist, dass er eine Öffnung (30) zwischen der Innen- und Außenseite der Maske (10) definiert, wobei die Außenseite der Maske (10) zu Umgebungsbedingungen hin offen ist, wobei der Ventilkörper (26) ein verformbares Ventilelement (22) stützt, das als Reaktion auf eine Druckdifferenz an dem Ventilelement (22) zwischen einer offenen und geschlossenen Bedingung relativ zu dem Ventilkörper (26) betätigbar ist,
wobei das Ventilelement (22) sowohl in einem ersten Betriebsmodus als Reaktion auf einen positiven Relativdruck auf der Innenseite, um ein Entweichen von Gas aus der Maske (10) zu dem Äußeren der Maske (10) zu ermöglichen, als auch in einem zweiten Betriebsmodus als Reaktion auf einen negativen Relativdruck im Inneren der Maske (10), um zu ermöglichen, dass Gas von der Außenseite in die Maske (10) gesaugt wird, öffenbar ist;
**dadurch gekennzeichnet, dass** das Ventilelement (22) im ersten Betriebsmodus im Wesentlichen unverformt ist und dass ein erster Abschnitt (22A) des Ventilelements (22) so angeordnet ist, dass er eine Lippe (36) des Ventilkörpers berührt, und ein zweiter Abschnitt (22B) des Ventilelements (22) so angeordnet ist, dass er in seiner geschlossenen Bedingung ein von der Lippe (36) versetztes Gebilde (40) berührt, sodass das Ventilelement (22) in der geschlossenen Bedingung verformt ist.

2. Atemmaske (10) nach Anspruch 1, wobei das Ventilelement (22) ein singulärer Körper aus elastischem oder flexiblem Material ist.

3. Atemmaske (10) nach Anspruch 1 oder 2, wobei das Ventilelement (22) ringförmig ist.

4. Atemmaske (10) nach einem vorhergehenden Anspruch, wobei das Ventilelement (22) in einer Ruhebedingung oder unverformten Bedingung flach oder planar ist.

5. Atemmaske (10) nach einem vorhergehenden Anspruch, umfassend ein Anschlussgebilde (17) zum Anschluss an eine Gasversorgung, wobei das Anschlussgebilde (17) einen Einlass in die Maske (10) umfasst, wobei der Ventilkörper (26) von dem Einlass beabstandet ist.

6. Atemmaske (10) nach einem vorhergehenden Anspruch, wobei der Ventilkörper (26) in einer Seitenwand (18) der Maske (10) bereitgestellt ist.

7. Atemmaske (10) nach einem vorhergehenden Anspruch, wobei sich das Ventilelement (22) im ersten Betriebsmodus von dem Maskenkörper (10) nach außen öffnet.

8. Atemmaske (10) nach einem vorhergehenden Anspruch, wobei das Ventilelement (22) von außerhalb der Maske (10) sichtbar ist.

9. Atemmaske (10) nach einem vorhergehenden Anspruch, wobei das Ventilelement (22) im zweiten Betriebsmodus verformt ist.

10. Atemmaske (10) nach Anspruch 9, wobei das Ventilelement (22) im zweiten Betriebsmodus von dem Maskenkörper (10) nach innen verformt ist.

11. Atemmaske (10) nach einem vorhergehenden Anspruch, wobei sich im zweiten Betriebsmodus nur der zweite Abschnitt (22B) des Ventilelements (22) öffnet.

12. Atemmaske (10) nach Anspruch 11, wobei sich das Ventilelement (22) als Reaktion auf den negativen Druck in dem zweiten Abschnitt (22B) verformt, um eine Strömung dort hindurch zu ermöglichen.

13. Atemmaske (10) nach einem vorhergehenden Anspruch, wobei die Größe des positiven Druckgradienten, der zum Öffnen des Ventils (20) im ersten Betriebsmodus erforderlich ist, geringer ist als die Größe des negativen Druckgradienten, der zum Öffnen des Ventils (20) im zweiten Betriebsmodus erforderlich ist.

## Revendications

1. Masque respiratoire (10) comportant un corps de soupape (26) formé en son sein de manière à définir une ouverture (30) entre les côtés intérieur et extérieur du masque (10), le côté extérieur du masque (10) étant ouvert aux conditions ambiantes, le corps de soupape (26) supportant un élément de soupape (22) déformable qui peut être actionné entre des états ouvert et fermé par rapport au corps de soupape (26) en réaction à une différence de pression à travers l'élément de soupape (22),
ledit élément de soupape (22) pouvant être ouvert à la fois dans un premier mode de fonctionnement en réaction à une pression relative positive sur le côté intérieur pour permettre l'échappement de gaz du masque (10) vers l'extérieur du masque (10) et dans un second mode de fonctionnement en réaction à une pression relative négative sur l'intérieur du masque (10) pour permettre l'aspiration de gaz dans le masque (10) depuis le côté extérieur ;
**caractérisé en ce que** l'élément de soupape (22) est sensiblement non déformé dans ledit premier mode de fonctionnement, et **en ce qu'**une première partie (22A) de l'élément de soupape (22) est agencée pour entrer en contact avec une lèvre (36) du corps de soupape et une seconde partie (22B) de l'élément de soupape (22) est agencée pour entrer en contact avec une partie conformée (40) décalée de la lèvre (36) dans son état fermé de sorte que l'élément de soupape (22) soit déformé dans ledit état fermé.

2. Masque respiratoire (10) selon l'une quelconque de la revendication 1, ledit élément de soupape (22) étant un corps singulier de matériau élastique ou souple.

3. Masque respiratoire (10) selon la revendication 1 ou 2, ledit élément de soupape (22) étant de forme annulaire.

4. Masque respiratoire (10) selon l'une quelconque des revendications précédentes, ledit élément de soupape (22) étant plat ou plan dans un état au repos ou non déformé.

5. Masque respiratoire (10) selon l'une quelconque des revendications précédentes, comprenant une partie conformée de raccord (17) destinée au raccordement à une alimentation de gaz, la partie conformée de raccord (17) comprenant une entrée dans le masque (10), ledit corps de soupape (26) étant espacé de l'entrée.

6. Masque respiratoire (10) selon l'une quelconque des revendications précédentes, ledit corps de soupape (26) étant pourvu dans une paroi latérale (18) du masque (10).

7. Masque respiratoire (10) selon l'une quelconque des revendications précédentes, ledit élément de soupape (22) s'ouvrant vers l'extérieur du corps de masque (10) dans le premier mode de fonctionnement.

8. Masque respiratoire (10) selon l'une quelconque des revendications précédentes, ledit élément de soupape (22) étant visible depuis l'extérieur du masque (10).

9. Masque respiratoire (10) selon l'une quelconque des revendications précédentes, ledit élément de soupape (22) étant déformé dans le second mode de fonctionnement.

10. Masque respiratoire (10) selon la revendication 9, ledit élément de soupape (22) étant déformé vers l'intérieur du corps de masque (10) dans le second mode de fonctionnement.

11. Masque respiratoire (10) selon l'une quelconque des revendications précédentes, seule ladite seconde partie (22B) de l'élément de soupape (22) s'ouvrant dans le second mode de fonctionnement.

12. Masque respiratoire (10) selon la revendication 11, ledit élément de soupape (22) se déformant en réaction à la pression négative dans la seconde partie (22B) afin de permettre l'écoulement à travers celle-ci.

13. Masque respiratoire (10) selon l'une quelconque des revendications précédentes, l'amplitude du gradient de pression positive requise pour ouvrir la soupape (20) dans le premier mode de fonctionnement étant inférieure à l'amplitude du gradient de pression négative requise pour ouvrir la valve (20) dans le second mode de fonctionnement.
